# FASCICULE DE BREVET EUROPEEN

(11) **EP 0 922 027 B1**
(45) Date de publication et mention de la délivrance du brevet: **25.09.2002**
(21) Numéro de dépôt: 97935660.7
(22) Date de dépôt: 31.07.1997
(51) Int. Cl.: C07D 201/16, C07D 201/08, C07C 253/34

(54) **PROCEDE DE PURIFICATION DE LACTAMES**
VERFAHREN ZUR REINIGUNG VON LACTAMEN
METHOD FOR PURIFYING LACTAMS

(30) Priorité: 02.08.1996 FR 9609973
(43) Date de publication de la demande: 16.06.1999
(73) Titulaire: RHODIA POLYAMIDE INTERMEDIATES, 69190 Saint-Fons (FR)
(72) Inventeur: GAYET, Hubert, F-69100 Villeurbanne (FR); LECONTE, Philippe, F-69330 Meyzieu (FR); PERRONA, Philippe, F-69390 CHARLY (FR)
(74) Mandataire: Esson, Jean-Pierre
(86) Numéro de dépôt international: FR9701426
(87) Numéro de publication internationale: WO98005636

(56) Documents cités:
- EP-A- 0 251 852
- DE-C- 850 746
- DE-C- 924 213
- US-A- 2 405 969
- US-A- 5 496 941
- CHEMICAL ABSTRACTS, vol. 68, no. 2, 8 janvier 1968 Columbus, Ohio, US; abstract no. 6128, TEMPLE, DIANA M.: "Liquid ion-exchange separation oe some physiologically active amines" XP002027598 & AUSTRALAS. J. PHARM. (1966), 47(559), S62-S64,

## Description

La présente invention concerne la purification de lactames par extraction liquide-liquide et/ou traitement par résine échangeuse d'ions.

Parmi les lactames les plus courants, le caprolactame est un composé très important, matière première à la base du polyamide 6 dont l'utilisation dans le monde est considérable.

Un procédé d'obtention du caprolactame, et par analogie des autres lactames, est de réaliser une hydrolyse cyclisante de l'aminonitrile correspondant, l'amino-6 capronitrile pour le caprolactame.

Une simple distillation du lactame ainsi obtenu n'est pas suffisante pour conférer audit lactame une pureté compatible avec la qualité requise pour les applications du polyamide correspondant. C'est le cas plus particulièrement en ce qui concerne le caprolactame pour les applications textiles du polyamide 6.

Il demeure notamment des quantités d'aminonitrile n'ayant pas été transformé encore trop importantes, ainsi que des quantités plus faibles, mais néanmoins excessives, d'autres sous-produits.

Le lactame et l'aminonitrile dont il provient, seraient théoriquement séparables par distillation. Cependant, il s'avère en pratique que lors du chauffage, il se forme probablement une certaine quantité d'un produit d'addition de ces deux composés, produit d'addition qui rétrograde ultérieurement en libérant à nouveau l'aminonitrile que l'on souhaitait séparer. C'est donc pourquoi une simple distillation ne permet pas une excellente séparation de l'aminonitrile et du lactame.

Le brevet WO-A-96/20923 préconise un procédé de purification du caprolactame comportant la succession suivante d'opérations. Tout d'abord le caprolactame est préparé par réaction de l'amino-6 capronitrile avec l'eau, puis les composés légers et les composés lourds du caprolactame brut sont séparés, ensuite le caprolactame issu de l'étape précédente est traité par l'hydrogène, en présence d'un catalyseur, à une température de 50-150°C et sous une pression de 1,5 à 250 bars pour donner un mélange A, qui est soit passé; sous forme d'une solution dans un solvant, sur une résine échangeuse d'ions à fonctions acides, soit distillé en présence d'acide sulfurique, enfin le mélange B1 ou B2 obtenu dans l'une des deux étapes précédentes est distillé en présence d'une base pour donner le caprolactame pur.

Lorsque l'on approfondit l'analyse de ce brevet, on constate que le procédé est très intimement lié à la purification du caprolactame préparé par hydrolyse en phase liquide. Cela ressort très nettement des exemples de réalisation de l'invention.

On observe également que le procédé de purification décrit est un procédé complexe comprenant de multiples étapes. Il apparaît également très coûteux et très vorace en énergie, puisque la première étape suivant la préparation du caprolactame consiste pratiquement à distiller l'ensemble des produits issus de la réaction. En effet, il est prévu de distiller les produits légers, c'est-à-dire ayant les plus faibles points d'ébullition et de séparer les produits lourds, c'est-à-dire ayant les points d'ébullition les plus élevés. Cela implique donc nécessairement la distillation de la totalité du caprolactame ayant par définition un point d'ébullition intermédiaire entre ceux des produits légers et des produits lourds.

La demanderesse a trouvé de manière inattendue par rapport à l'enseignement du brevet WO-A-96/20923, que les lactames préparés par hydrolyse cyclisante des aminonitriles en phase vapeur, pouvaient être obtenus avec une pureté du même ordre que celles des lactames préparés en phase liquide, en utilisant un procédé de purification ne comportant pas la distillation préalable de tous les constituants du mélange réactionnel issu de la réaction d'hydrolyse.

La seule opération préalable au présent procédé de purification est l'élimination de l'ammoniac formé, cette élimination consistant le plus souvent en une distillation dudit ammoniac.

La présente invention consiste en un procédé de purification d'un lactame provenant de l'hydrolyse cyclisante en phase vapeur d'un aminonitrile aliphatique, caractérisé en ce que sans distillation préalable du lactame et après avoir éliminé la majeure partie de l'ammoniac qu'il comporte, on soumet ledit lactame à une extraction liquide-liquide à l'aide d'un solvant comprenant un solvant à caractère acide et/ou on met en contact ledit lactame avec une résine échangeuse de cations.

Le lactame mis en oeuvre dans le présent procédé est choisi parmi ceux qui sont obtenus par hydrolyse cyclisante en phase vapeur d'un aminonitrile aliphatique de formule générale (I) :

N≡C―R―NH₂ (I)

dans laquelle R représente un radical alkylène ayant de 3 à 12 atomes de carbone.

Parmi les lactames, les plus importants sont ceux qui servent de matière première pour la préparation des polyamides 4, 5, 6 et 10 et qui sont obtenus à partir des aminonitriles de formule (I), dans laquelle le symbole R représente un radical alkylène linéaire ayant 3, 4, 5 ou 9 atomes de carbone.

Comme indiqué précédemment, le caprolactame dont la polymérisation fournit le polyamide 6, qui est préparé à partir de l'amino-6 capronitrile (ou epsilon-capronitrile) et contient donc une certaine quantité de ce dernier composé, est le lactame préférentiellement mis en oeuvre dans le procédé de l'invention.

A titre d'illustration non limitative du procédé de préparation de lactame par hydrolyse cyclisante en phase vapeur d'aminonitriles de formule (I), on peut se référer par exemple aux brevets EP-A-0 659 741, US 2 357 484 ou WO-A-96/22974.

Le lactame à purifier est de préférence sous forme d'une solution aqueuse. La concentration en lactame d'une telle solution est en général de 20 % à 80 % en poids par poids. L'aminonitrile non transformé représente le plus souvent jusqu'à 15 % du poids du lactame et de préférence de 0,1 % à 10 % de ce poids.

L'ammoniac qui se forme lors de l'hydrolyse cyclisante de l'aminonitrile en quantité molaire égale à celle du lactame, se trouve encore partiellement dans la solution dudit lactame. Son élimination est effectuée généralement par distillation.

Cette distillation peut être réalisée par chauffage de la solution de caprolactame jusqu'à une température en pied de 100°C à 190°C, de préférence de 140°C à 160°C, sous une pression absolue de 1 à 10 bars, sans que ces valeurs soient considérées comme critiques. Le choix des conditions opératoires est fait de telle façon que l'on élimine ainsi plus de 50 % et de préférence plus de 90 % de l'ammoniac tout en ne distillant qu'une partie relativement mineure de l'eau, afin de ne pas obtenir une solution ayant une concentration en lactame supérieure à 80 % en poids par poids.

Le solvant à caractère acide utilisé dans l'extraction liquide-liquide peut être en particulier acide carboxylique ou un hydrogénophosphate d'alkyle et plus particulièrement un hydrogénophosphate de dialkyle. Ainsi on peut utiliser un hydrogénophosphate de dialkyle dans la formule duquel les groupes alkyles, linéaires ou ramifiés et de préférence identiques, ont de 1 à 12 atomes de carbone. Parmi ces composés, l'hydrogénophosphate de di(2-éthyl hexyle) est le plus souvent utilisé, notamment en raison de sa disponibilité commerciale à grande échelle. L'hydrogénophosphate de dialkyle peut contenir une certaine proportion d'hydrogénophosphate de monoalkyle correspondant, ce composé étant également formé lors de la préparation de l'hydrogénophosphate de dialkyle. Généralement cette proportion d'hydrogénophosphate de monoalkyle n'excède pas 20 % et de préférence est inférieure ou égale à 10 % en poids par poids. Comme acide carboxylique, on peut citer par exemple l'acide heptanoïque, l'acide éthyl-2 hexanoïque.

Lorsque le solvant à caractère acide est relativement visqueux, il peut être utile de le mettre en oeuvre pour l'extraction liquide-liquide en mélange avec un autre liquide organique que l'on appellera diluant dans le présent texte. Ce diluant doit dissoudre peu de lactame dans les conditions d'utilisation. La solubilité du lactame dans le diluant est généralement inférieure ou égale 200 grammes par litre à 25°C et de préférence inférieure ou égale à 100 grammes par litre.

La proportion de diluant dans le liquide utilisé pour réaliser l'extraction liquide-liquide du procédé de l'invention varie habituellement de 0 % à 80 % et de préférence de 10% à 60 % en poids par poids.

L'extraction s'effectue selon les méthodes connues comme par exemple la circulation à contre-courant de la solution de lactame et du solvant d'extraction, le terme solvant d'extraction englobant le solvant à caractère acide seul ainsi que ses mélanges avec un diluant.

On dispose classiquement en extraction liquide/liquide de contacteurs qui sont, soit étagés de type mélangeurs-décanteurs, soit différentiels de type colonne gravitaires. Cette deuxième famille se divise en deux sous-familles : les colonnes non-agitées et les colonnes mécaniquement agitées. Dans le premier cas, il s'agit essentiellement de colonnes garnies ou à plateaux perforés, dans le deuxième cas il s'agit essentiellement de colonnes pulsées ou agitées.

Le choix de la technologie permet d'obtenir le meilleur compromis entre son coût, en investissement et en fonctionnement, et son efficacité en prenant en compte différents critères tels que le rapport des phases solvant/produit à purifier, l'efficacité d'extraction, l'encombrement au sol de l'appareillage, le volume total de liquide dans l'appareil, la capacité à traiter des solutions contenant des impuretés en suspension, corrosion des matériaux utilisés.

Le rapport volumique entre la solution aqueuse de lactame soumise à l'extraction et le solvant d'extraction varie habituellement entre 1/5 et 5/1. De préférence ce rapport se situe entre 211 et 112.

La température à laquelle est conduite l'extraction peut se situer notamment entre 10°C et 90°C. On opère de préférence à une température de 20°C à 80°C.

Les résines échangeuses de cations sont des résines polymériques comportant des fonctions à caractère acide. Ce sont généralement, mais non exclusivement des résines portant des fonctions acides sulfoniques ou acides carboxyliques. On peut également utiliser des résines échangeuses de cations à caractère complexant qui seront englobées dans le présent texte dans les résines à fonction acides. Ces résines échangeuses de cations à caractère complexant possèdent généralement des fonctions de type imidodiacétiques ou aminophosphoniques. Ces produits, moins couramment utilisés et d'un prix plus élevé que les autres résines échangeuses de cations, sont également plus sélectifs et plus efficaces, en raison de la combinaison des propriétés d'échanges d'ions et de complexation.

Les résines sulfoniques sont de deux types : les résines à structure gel pour lesquelles la porosité interne des billes est naturelle, les résines à structure macroporeuse pour lesquelles la porosité interne est artificielle et est déterminée par la présence de canaux (diamètre des pores allant jusqu'à 150 nm).

Les résines macroporeuses sont plus fortement réticulées que les résines de type gel. Le taux de réticulation correspond au pourcentage en poids de divinylbenzène dans le monomère.

De manière générale, l'augmentation du taux de réticulation entraîne une augmentation de la tenue en milieu oxydant, de la rigidité des billes, donc de la résistance à l'attrition et aux tensions osmotiques (pression dans les pores due au changement de taille des ions fixés et de leur couche hydratée), de la résistance au transfert interne liée à une structure plus dense (vitesse de circulation des ions dans la porosité plus faible), de l'affinité et de la sélectivité de la résine pour les diverses espèces ioniques. Par contre, on observe une diminution de la capacité totale d'échange et de l'efficacité de l'élution.

Dans le cadre de la présente invention, les résines échangeuses de cations à fonctions acides sulfoniques et à structure macroporeuse, conviennent particulièrement bien. En effet, cette structure confère aux billes de résine une plus grande solidité lorsque la nature du milieu change entre l'adsorption et les autres phases du cycle : milieu à caractère organique pour l'adsorption, milieu aqueux pour les lavages et l'élution.

La solution aqueuse de lactame peut être mise en contact avec la résine échangeuse de cations sans addition d'un tiers solvant. Si nécessaire, elle peut être diluée par de l'eau.

A titre indicatif, la solution de lactame traitée par une résine échangeuse de cations comprend généralement de 10 % à 90 % de composés dissous en poids par rapport au poids de la solution et le plus souvent de 20 % à 80 %.

Pour une mise en oeuvre industrielle, les traitements sur résine sont réalisés de manière très générale en colonne et de préférence en utilisant au minimum deux colonnes fonctionnant en marche alternée.

Le cycle de traitement sur lit de résine est généralement composé d'une étape d'adsorption, d'une étape de rinçage après adsorption, d'une étape de régénération du lit par mise en contact avec une solution d'un acide minéral protonique et d'une étape de rinçage après élution du flux de régénération.

L'étape d'adsorption consiste dans l'échange de cations (protons et ammonium) entre la résine et la solution de lactame à traiter (solution alimentaire). L'écoulement de la solution dans le lit peut être réalisé soit de haut en bas (procédé à lit bloqué), soit de bas en haut (procédé à lit flottant). L'opération est interrompue quand le front de saturation atteint l'extrémité du lit de résine.

Le rinçage après adsorption a pour principal objectif d'éviter la pollution de l'éluat par la solution alimentaire contenue dans le lit de résine en fin d'adsorption. Il s'effectue généralement à l'eau et peut être réalisé en deux étapes successives. La première étape du rinçage consiste en un déplacement de la solution alimentaire contenue dans le volume interstitiel du lit en fin d'adsorption, afin de la récupérer pour sa valorisation. La quantité d'eau généralement nécessaire est environ égale au volume interstitiel du lit (ou porosité externe du lit) dans l'hypothèse où la distribution du liquide en entrée est correcte et le lit ne présente pas de passage préférentiel. La deuxième étape du rinçage consiste dans le rinçage proprement dit de la résine pour débarrasser les pores des billes de la résine des traces de la solution alimentaire. La quantité d'eau est alors dépendante de la nature de la résine (porosité interne) ainsi que des conditions opératoires (notamment la vitesse d'écoulement de l'eau dans le lit). Globalement le transfert de matière entre les pores et la solution de rinçage est limité par la cinétique de diffusion interne dans les billes.

La régénération de la résine consiste dans le traitement (notamment sous forme de percolation) à l'aide d'une solution d'acide minéral protonique tel que par exemple l'acide sulfurique, l'acide nitrique, l'acide chlorhydnque. Cette solution est généralement concentrée. Elle contient par exemple de 1 à 3 équivalents H⁺ par litre. Cette opération permet la régénération des sites actifs sous forme de protons et elle entraîne selon l'acide utilisé la formation d'un éluat riche en sulfates, nitrates ou chlorures des amines retenues sur la résine, notamment de l'aminocapronitrile.

Le sens d'écoulement du liquide dans cette étape de régénération peut être le même que celui de l'étape d'adsorption (régénération à co-courant) ou être inverse (régénération à contre-courant). Ce deuxième type de régénération présente généralement une efficacité supérieure et est le plus souvent préféré. La régénération à co-courant se met en oeuvre avec un sens de passage du liquide dans le lit de résine aussi bien ascendant que descendant. Dans le premier cas, le lit doit être bloqué (par exemple par une légère pression d'un gaz inerte au-dessus du lit) afin d'éviter la fluidisation des billes de résine. Ce soulèvement du lit aurait pour conséquence, d'une part de réduire l'efficacité de l'élution, d'autre part de mélanger les différentes couches du lit de résine plus ou moins bien régénérées. Dans le second cas, il n'est pas nécessaire de prévoir un blocage du lit. Par contre un tel blocage (mécanique de préférence) est nécessaire tors de l'étape d'adsorption qui s'effectue alors de bas en haut. Un tel blocage mécanique se fait par exemple par l'intermédiaire d'un plafond crépiné.

L'étape de rinçage après régénération peut être composée, comme pour l'étape de rinçage après l'adsorption, de deux étapes successives : déplacement du volume interstitiel et rinçage des billes de résine pour éliminer les dernières traces d'acide contenues dans la porosité des billes. Le sens du rinçage est le même que celui de la régénération.

Bien que le procédé de purification du lactame décrit précédemment conduise déjà à un lactame de pureté correcte, il est préférable pour la plupart des applications dudit lactame, et tout particulièrement du caprolactame, de faire précéder ou de compléter l'étape d'extraction liquide/liquide et/ou l'étape de passage sur résine acide par une étape d'hydrogénation des composés de la solution de lactame, qui comportent des insaturations. Ces composés sont principalement les composés qui possèdent des fonctions nitrile, comme l'aminonitrile de départ ou certains sous-produits présentant des fonctions imines ou des doubles liaisons carbone-carbone.

L'hydrogénation est préférentiellement réalisée avant le passage sur résine et/ou l'extraction liquide-liquide, ces derniers traitements permettant ainsi d'éliminer les amines formées par hydrogénation.

Cette étape d'hydrogénation est généralement effectuée à une température de 50°C à 150°C, sous une pression en température de 1 à 100 bars et en présence d'un catalyseur d'hydrogénation.

Comme catalyseur d'hydrogénation, on peut citer les catalyseurs à base de nickel de Raney et/ou de cobalt de Raney, comportant éventuellement, mais préférentiellement, un élément dopant choisi parmi les éléments des groupes IVb, Vlb, Vllb et VIII de la classification périodique des éléments telle que publiée dans Handbook of Chemistry and Physics, 51st Edition (1970-1971).

Le catalyseur à base de nickel de Raney et/ou de cobalt de Raney utilisé dans le procédé peut donc comporter, outre le nickel ou le cobalt et les quantités résiduelles du métal éliminé de l'alliage d'origine lors de la préparation du catalyseur, c'est-à-dire généralement l'aluminium, un ou plusieurs autres éléments dopants, tels que par exemple le chrome, le titane, le molybdène, le tungstène, le fer, le zinc.

Parmi ces éléments dopants le chrome et/ou le fer et/ou le titane sont considérés comme les plus avantageux. Ces dopants représentent habituellement, en poids par poids de nickel ou de cobalt, de 0 % à 15 % et de préférence de 0,1 % à 10 %.

Le catalyseur peut aussi être constitué par un métal, qui est généralement un métal du groupe VIII de la classification périodique des éléments tel que le ruthénium, le rhodium, l'iridium, l'osmium, le platine, le palladium, le nickel ou le cobalt, déposé sur un support qui est généralement un oxyde métallique tel que les alumines, les silices, les aluminosilicates, le dioxyde de titane, l'oxyde de zirconium, l'oxyde de magnésium.

Dans les catalyseurs métalliques supportés, le métal représente généralement de 0,1 à 80 % du poids du support et préférentiellement de 0,5 à 50 %.

L'étape d'hydrogénation peut être complétée ou être remplacée par une étape d'oxydation. De préférence, l'étape d'oxydation sera une étape de remplacement de l'étape d'hydrogénation plutôt qu'une étape additionnelle.

L'oxydation peut être effectuée à l'aide de peroxyde d'hydrogène, d'ozone ou de sels oxydants comme le permanganate de potassium.

De préférence l'oxydation est réalisée avec le peroxyde d'hydrogène en milieu basique, notamment en milieu contenant un hydroxyde de métal alcalin.

Lorsque le procédé de l'invention çomporte une étape d'oxydation, celle-ci peut précéder ou suivre l'étape d'extraction liquide/liquide et/ou l'étape de passage sur résine acide, mais elle est réalisée de préférence postérieurement à l'étape d'extraction liquide/liquide et/ou l'étape de passage sur résine acide.

Le procédé selon l'invention est complété de manière tout à fait préférentielle par l'isolement par distillation du caprolactame à partir de sa solution aqueuse ayant subi les différentes étapes de purification détaillées précédemment.

Cette distillation est effectuée dans les conditions habituellement mises en oeuvre pour le caprolactame. Ainsi, elle sera conduite de préférence sous une pression inférieure à la pression atmosphérique, afin d'éviter de soumettre le caprolactame à des températures élevées pendant une durée trop longue. La pression absolue sera le plus souvent comprise entre 100 Pa et la pression atmosphérique et de préférence comprise entre 100 Pa et 20 kPa. Généralement il est avantageux de ne pas dépasser une température d'environ 150°C dans le bouilleur lors de la distillation. De manière préférentielle, la distillation sera effectuée en présence d'une base. La base peut être choisie parmi les hydroxydes de métal alcalin, les hydroxydes de métal alcalino-terreux, les carbonates de métal alcalin et les carbonates de métal alcalino-terreux. Le plus souvent la base mise en oeuvre sera l'hydroxyde de sodium.

L'appareillage utilisé pour cette étape de distillation est l'appareillage habituellement utilisé. Il sera avantageux d'employer une colonne de distillation possédant un nombre relativement important de plateaux théoriques, de préférence au moins 10 plateaux théoriques.

La quantité de base mis en oeuvre, exprimée en poids par poids de caprolactame se situe généralement entre 0,01 % et 2 %.

Les exemples qui suivent illustrent l'invention.

### EXEMPLE 1

Un mélange brut provenant d'une réaction d'hydrolyse cyclisante de l'amino-6 capronitrile (ACN), préalablement évaporé pendant 30 min à 90°C pour éliminer l'ammoniac qu'il contenait, présente la composition suivante :
- ACN : 0,870 % en poids par poids
- caprolactame (CPL) : 59,10 % en poids par poids
- sous-produits : 3,17 % en poids par poids
- eau : complément à 100 % en poids.

Un échantillon de 299 g de ce mélange est mis en contact avec 600 g d'un mélange comportant 30 % en poids d'hydrogénophosphate de di(2-éthylhexyle) et 70 % en poids de cyclohexane à une température de 50°C. Après mise en équilibre, la dispersion est décantée et un échantillon est prélevé dans chacune des deux phases, afin de suivre la répartition de l'ACN et du CPL.

L'opération est répétée avec la phase aqueuse provenant de la première extraction (ou premier étage de l'extraction), avec 366 g du même mélange d'hydrogénophosphate de di(2-éthylhexyle) et de cyclohexane, mais à une température de 34°C (deuxième étage de l'extraction).

Les résultats obtenus dans les deux étages d'extraction sont rassemblés dans le tableau 1 ci-après.

Le coefficient m correspond au rapport des teneurs en masse du composé considéré, dans les deux phases en équilibre.

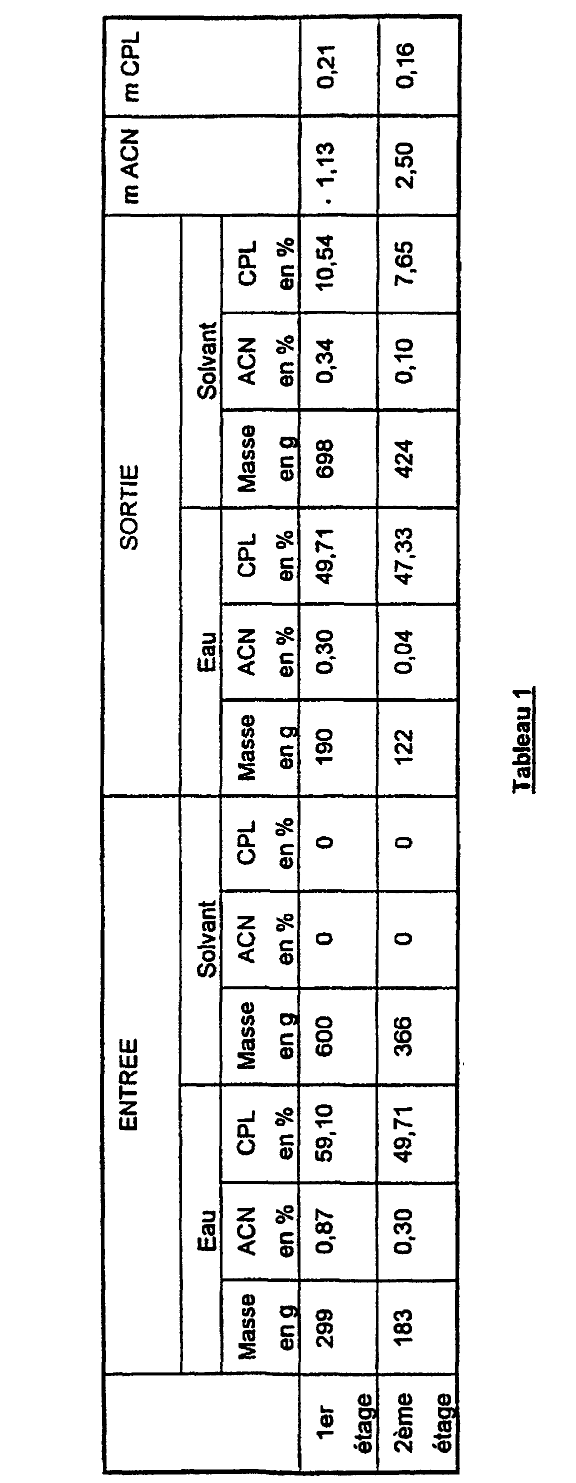

### EXEMPLES 2 A 5

Un mélange brut provenant d'une réaction d'hydrolyse cyclisante de l'amino-6 capronitrile (ACN), préalablement évaporé pendant 30 min à 90°C pour éliminer l'ammoniac qu'il contenait, présente la composition suivante :
- ACN : 5,30 % en poids par poids
- caprolactame (CPL) : 56,90 % en poids par poids
- sous-produits légers (éluant en chromatographie avant le CPL) : 0,14 % en poids par poids
- sous-produits lourds (éluant après le CPL) : 0,58 % en poids par poids
- eau : complément à 100 % en poids.

Ce mélange alimentaire est traité avec une quantité de résine sulfonique macroporeuse (de marque Amberlite 252 H®) indiquée dans le tableau 2 ci-après. Cette résine, commercialisée sous forme protonique, est préalablement lavée à l'eau et à l'éthanol.

Deux rapports volumiques mélange alimentaire/résine humide sont mis en oeuvre Ce rapport est déterminé de telle façon que le rapport ( exprimé en équivalents) entre la charge ionique de la solution (en tenant compte exclusivement de l'ACN) et le nombre théorique de sites actifs de la résine sulfonique soit voisin de 1 (exemple 2) et de 0,3 (exemple 3).

Le traitement consiste à mettre en contact, dans un réacteur sous agitation, un volume déterminé de mélange alimentaire et une quantité fixée de résine humide, à 80°C. Après mise au repos, un échantillon de liquide est prélevé et un dosage par chromatographie en phase gazeuse est effectué, pour déterminer le partage des espèces entre la solution et la résine : teneurs en poids/poids (p/p) de la solution en fin de traitement

Les mêmes essais sont réalisés avec un mélange alimentaire plus dilué de composition suivante :
- ACN : 3,30 % en poids par poids
- caprolactame (CPL) : 35,70 % en poids par poids
- sous-produits légers (éluant avant le CPL) : 0,09 % en poids par poids
- sous-produits lourds (éluant après le CPL) : 0,36 % en poids par poids
- eau : complément à 100 % en poids.

Le tableau 2 ci-après rassemble les principales caractéristiques des différents exemples ainsi que les résultats obtenus.

**Tableau 2**

| Exemples | Exemple 2 | | Exemple 3 | | Exemple 4 | | Exemple 5 | |
|---|---|---|---|---|---|---|---|---|
| Rapport solution/résine (en éq) | 1,31 | | 0.26 | | 1,23 | | 0,27 | |
| volume solution | 150,7 ml | | 100,9 ml | | 150,8 ml | | 100,5 ml | |
| volume résine | 30,0 ml | | 100,0 ml | | 20,0 ml | | 60,0 ml | |
| Teneurs en % p/p | début | fin | début | fin | début | fin | début | fin |
| eau | 36.0 | 45,0 | 36,0 | 43,8 | 59,9 | 64,3 | 59,9 | 64,2 |
| légers | 0,14 | 0,02 | 0,14 | 0,02 | 0,09 | 0,05 | 0,09 | 0,02 |
| ACN | 5,30 | 2,40 | 5,30 | 0,01 | 3,30 | 1,55 | 3,30 | 0,00 |
| CPL | 56,90 | 52,40 | 56,90 | 56,90 | 35,70 | 34,00 | 35,70 | 35,70 |
| lourds | 0,58 | 0,39 | 0,58 | 0,11 | 0,36 | 0,22 | 0,36 | 0,07 |

La teneur en eau dans ces essais n'a pas d'influence nette sur le comportement de la résine vis-à-vis des différents composés.

Avec un rapport solution/résine de 0,3 environ, on atteint une teneur en ACN inférieure ou égale à 0,01 %;

La fixation du CPL sur la résine est négligeable, voire nulle tandis que les légers et les lourds présentent une affinité pour la résine relativement importante.

### EXEMPLE 6

Un mélange brut (contenant 60 % en poids de caprolactame) provenant d'une réaction d'hydrolyse cyclisante de l'amino-6 capronitrile (ACN) est chauffé sous pression atmosphérique, à une température commençant à 20°C et allant jusqu'à 111°C dans le bouilleur pendant quelques heures pour éliminer l'ammoniac qu'il contenait.

Après cette opération, la solution contient 77,6 % en poids par poids de produits organiques dont la répartition pondérale est la suivante : 97,42 % de caprolactame, 1,71 % en poids par poids d'ACN et 0,87 % en poids par poids de divers autres sous-produits.

Ce mélange est ensuite passé avec un débit de 3,1 litres/heure sur une résine sulfonique macroporeuse (de marque DUOLITE A 252 H® de la Société Rohm et Haas)

Le passage sur résine provoque la décoloration de la solution aqueuse de caprotactame : elle passe d'une couleur orangée à une couleur jaune clair.

Environ 85 % des sous-produits ont été retenus sur la résine.

La teneur pondérale en ACN est passée à 0,0006 % par rapport au caprolactame, tandis que la pureté en caprolactame est passée à 99,8 %, compte non tenu de l'eau présente.

La solution de caprolactame ainsi traitée est distillée à l'aide d'une colonne à garnissage présentant environ 20 plateaux théoriques, en présence de 0,2 % de NaOH par rapport au caprolactame, sous une pression réduite progressivement jusqu'à une valeur finale de 650 Pa, la température dans le bouilleur atteignant 145°C au maximum pendant la distillation. La première fraction de tête contient l'eau et l'essentiel des sous-produits légers restants. Les fractions suivantes ont un titre en caprolactame supérieur à 99,996 % (dosage par chromatographie en phase gazeuse).

Le caprolactame distillé répond aux spécifications pour la préparation de polyamide 6:
- indice de permanganate (selon norme ISO 8660) : 3,24 (spécification < 5)
- bases libres : < 0,06 milliéquivalent (meq)/kg de CPL (spécification < 0,1)
- bases volatiles (selon norme ISO 8661) : 0,45 meq/kg (spécification < 0,5)
- absorbance UV à 290 nm (selon norme ISO 7059) : 0,047 (spécification < 0,05).

### EXEMPLE 7

Environ 8 kg d'un mélange brut (contenant 57 % en poids de caprolactame) provenant d'une réaction d'hydrolyse cyclisante de l'amino-6 capronitrile (ACN) sont chauffés sous pression atmosphérique, à une température commençant à 20°C et allant jusqu'à 111°C dans le bouilleur pendant quelques heures pour éliminer l'ammoniac.

Après cette opération, la solution contient 67 % en poids par poids de produits organiques dont la répartition pondérale est la suivante : 93,09 % de caprolactame, 6,19 % en poids par poids d'ACN et 0,72 % en poids par poids de divers autres sous-produits.

Les bases libres représentent 706 meq/kg, la valeur de permanganate est de 93 et les bases volatiles représentent 620 meq/kg.

La valeur de permanganate est une caractéristique différente de l'indice de permanganate. Elle correspond au nombre de millilitres de solution de permanganate de potassium 0,2 N consommés par kilogramme de caprolactame en milieu sulfurique.

On réalise une hydrogénation en continu sur une partie de la solution de caprolactame. On charge 670 g de cette solution dans un autoclave agité de 1,31 permettant d'opérer en continu, ainsi que 30 g de Ni de Raney contenant 1,7 % de Cr et 0,8 mol de KOH/kg de Ni.

L'autoclave est chauffé à 80°C sous une pression de 20 bar d'hydrogène, puis on alimente la solution de caprolactame à raison de 500 g/h et une solution aqueuse 1 N de KOH à raison de 29 g/h.

La répartition pondérale en produits organiques de l'hydrogénat ainsi obtenu est la suivante : 92,89 % de caprolactame, 5,8 % d'hexaméthylènediamine, 0,01 % d'ACN et 1,3 % de divers autres sous-produits.

Les bases libres représentent 1446 meq/kg, la valeur de permanganate est de 54 et les bases volatiles représentent 116 meq/kg.

L'hydrogénat est ensuite passé sur 4,4 litres de la résine définie dans l'exemple 6 et dans les mêmes conditions.

La répartition pondérale en produits organiques de la solution après passage sur résine est la suivante : 99,92 % de caprolactame, 0,08 % pour l'ensemble des autres sous-produits.

Les bases libres représentent 53 meq/kg, la valeur de permanganate est de 18 et les bases volatiles représentent 4 meq/kg.

La solution de caprolactame ainsi traitée est distillée en continu en trois étapes à raide d'une colonne à garnissage présentant environ 45 plateaux théoriques, en présence de 0,2 % de NaOH par rapport au caprolactame, sous une pression réduite : tout d'abord déshydratation de la solution et récupération du caprolactame en pied, puis étêtage du caprolactame et enfin distillation du caprolactame lui-même.

Les conditions de la distillation sont les suivantes : pression d'environ 2000 Pa en pied ; température dans le bouilleur atteignant 145°C au maximum pendant la distillation.

Le caprolactame distillé a une pureté de 99,995 % et il répond aux spécifications pour la préparation de polyamide 6 :
- indice de permanganate (selon norme ISO 8660) : 2,9
- bases libres : 0,05 meq/kg
- bases volatiles (selon norme ISO 8661) : 0,18 meq/kg
- absorbance UV à 290 nm (selon norme ISO 7059): 0,024.

### EXEMPLE 8

On traite comme dans l'exemple 7 environ 10 kg du même mélange brut contenant 57 % en poids de caprolactame, pour en éliminer l'ammoniac.

La solution de caprolactame est ensuite passée sur 2 litres de la résine définie dans l'exemple 6 et dans les mêmes conditions.

La répartition pondérale en produits organiques de la solution après passage sur résine est la suivante : 99,29 % de caprolactame, 0,22 % d'ACN et 0,49 % pour l'ensemble des autres sous-produits.

Les bases libres représentent 258 meq/kg, la valeur de permanganate est de 95 et les bases volatiles représentent 48 meq/kg.

La solution obtenue après passage sur résine (53,1 % en poids de caprolactame) est oxydée dans les conditions suivantes. On ajoute 107 g de solution aqueuse à 30 % de soude, puis on chauffe à 50°C sous agitation. On coule ensuite 53,2 g de solution aqueuse à 30 % de peroxyde d'hydrogène et on maintient le mélange à 50°C sous barbotage d'azote pendant 2 heures 30.

La répartition pondérale en produits organiques de l'oxydat ainsi obtenu est la suivante : 99,6 % de caprolactame, 0,07 % d'ACN et 0,33 % de divers autres sous-produits.

Les bases libres représentent 238 meq/kg, la valeur de permanganate est de 180 et les bases volatiles représentent 54 meq/kg.

Cet oxydat est ensuite distillé en continu avec le même appareillage et dans les mêmes conditions que pour l'exemple 7.

Le caprolactame distillé a une pureté de 99,99 % et il répond aux spécifications pour la préparation de polyamide 6 :
- indice de permanganate (selon norme ISO 8660) : 3,4
- bases libres : 0,07 meq/kg
- bases volatiles (selon norme ISO 8661) : 0,25 meq/kg
- absorbance UV à 290 nm (selon norme ISO 7059) : 0,032.

## Revendications

1. Procédé de purification d'un lactame provenant de l'hydrolyse cyclisante en phase vapeur d'un aminonitrile aliphatique, **caractérisé en ce que** sans distillation préalable du lactame et après avoir éliminé la majeure partie de l'ammoniac qu'il comporte, on soumet ledit lactame à une extraction liquide-liquide à l'aide d'un solvant comprenant un solvant à caractère acide et/ou on met en contact ledit lactame avec une résine échangeuse de cations et **en ce que** le lactame mis en oeuvre est choisi parmi ceux qui sont obtenus par hydrolyse cyclisante en phase vapeur d'un aminonitrile aliphatique de formule générale (I):
N≡C―R―NH₂ (I)
dans laquelle R représente un radical alkylène ayant de 3 à 12 atomes de carbone.

2. Procédé selon la revendication 1, **caractérisé en ce que** le lactame mis en oeuvre est choisi parmi ceux qui sont obtenus par hydrolyse cyclisante en phase vapeur d'un aminonitrile aliphatique de formule générale (I) :
N≡C―R―NH₂ (I)
dans laquelle R représente un radical alkylène linéaire ayant 3, 4, 5 ou 9 atomes de carbone.

3. Procédé selon l'une des revendications 1 ou 2, **caractérisé en ce que** le lactame préférentiellement mis en oeuvre est le caprolactame préparé à partir de l'amino-6 capronitrile.

4. Procédé selon l'une des revendications 1 à 3, **caractérisé en ce que** le lactame à purifier est sous forme d'une solution aqueuse dont la concentration en lactame est de 20 % à 80 % en poids par poids.

5. Procédé selon l'une des revendications 1 à 4, **caractérisé en ce que** l'aminonitrile non transformé représente jusqu'à 15 % du poids du lactame et de préférence de 0,1 % à 10 % de ce poids.

6. Procédé selon l'une des revendications 1 à 5, **caractérisé en ce que** le solvant à caractère acide utilisé dans l'extraction liquide-liquide est un hydrogénophosphate d'alkyle et de préférence un hydrogénophosphate de dialkyle.

7. Procédé selon l'une des revendications 1 à 6, **caractérisé en ce que** le solvant à caractère acide utilisé dans l'extraction liquide-liquide est un hydrogénophosphate de dialkyle dans la formule duquel les groupes alkyles, linéaires ou ramifiés et de préférence identiques, ont de 1 à 12 atomes de carbone.

8. Procédé selon l'une des revendications 1 à 7, **caractérisé en ce que** le solvant à caractère acide utilisé dans l'extraction liquide-liquide est l'hydrogénophosphate de di(2-éthyl hexyle).

9. Procédé selon l'une des revendications 1 à 8, **caractérisé en ce que** le solvant à caractère acide est mis en oeuvre en mélange avec un autre liquide organique appelé diluant, la solubilité du lactame dans le diluant étant inférieure ou égale 200 grammes par litre à 25°C et de préférence inférieure ou égale à 100 grammes par litre.

10. Procédé selon l'une des revendications 1 à 9, **caractérisé en ce que** la proportion de diluant dans le liquide utilisé pour réaliser l'extraction liquide-liquide du procédé de l'invention varie de 0 % à 80 % et de préférence de 10% à 60 % en poids par poids.

11. Procédé selon l'une des revendications 1 à 10, **caractérisé en ce que** le rapport volumique entre la solution aqueuse de lactame soumise à l'extraction et le solvant d'extraction varie entre 1/5 et 5/1 et de préférence entre 2/1 et 1/2.

12. Procédé selon l'une des revendications 1 à 11, **caractérisé en ce que** la température à laquelle est conduite l'extraction se situe entre 10°C et 90°C et est de préférence de 20°C à 80°C.

13. Procédé selon l'une des revendications 1 à 5, **caractérisé en ce que** la résine échangeuse de cations est choisie parmi les résines polymériques comportant des fonctions à caractère acide, de préférence parmi les résines portant des fonctions acides sulfoniques ou acides carboxyliques) et les résines polymériques à caractère complexant, de préférence comportant des fonctions imidoacétiques ou aminophosphoniques.

14. Procédé selon la revendication 13, **caractérisé en ce que** la résine échangeuse de cations est choisie parmi les résines échangeuses de cations à fonctions acides sulfoniques et à structure macroporeuse.

15. Procédé selon l'une des revendications 13 ou 14, **caractérisé en ce que** la solution de lactame traitée par une résine échangeuse de cations comprend de 10 % à 90 % de composés dissous en poids par rapport au poids de la solution et de préférence de 20 % à 80 %.

16. Procédé selon l'une des revendications 13 à 15, **caractérisé en ce que** le traitement sur résine est réalisé en colonne et **en ce qu'**il comprend un cycle composé d'une étape d'adsorption, d'une étape de rinçage après adsorption, d'une étape de régénération du lit par mise en contact avec une solution d'un acide minéral protonique et d'une étape de rinçage après élution du flux de régénération.

17. Procédé selon l'une des revendications 13 à 16, **caractérisé en ce que** la régénération de la résine est réalisée à l'aide d'une solution d'acide minéral protonique tel que l'acide sulfurique, l'acide nitrique, l'acide chlorhydrique.

18. Procédé selon l'une des revendications 1 à 17, **caractérisé en ce que** l'étape d'extraction liquide/liquide et/ou l'étape de passage sur résine acide est précédée ou est complétée par une étape d'hydrogénation.

19. Procédé selon la revendication 18, **caractérisé en ce que** l'étape d'hydrogénation est conduite en présence d'un catalyseur choisi parmi les catalyseurs à base de nickel de Raney et/ou de cobalt de Raney, comportant préférentiellement un élément dopant choisi parmi les éléments des groupes IVb, Vlb, Vllb et VIII de la classification périodique, ou les catalyseurs constitués par un métal du groupe VIII de la classification périodique des éléments tel que le ruthénium, le rhodium, l'iridium, l'osmium, le platine, le palladium, le nickel ou le cobalt, déposé sur un support.

20. Procédé selon l'une des revendications 1 à 19, **caractérisé en ce que** l'étape d'extraction liquide/liquide et/ou l'étape de passage sur résine acide est précédée ou est suivie par une étape d'oxydation.

21. Procédé selon la revendication 20, **caractérisé en ce que** l'étape d'oxydation est postérieure à l'étape d'extraction liquide/liquide et/ou l'étape de passage sur résine acide et est effectuée à l'aide de peroxyde d'hydrogène, d'ozone ou de sels oxydants comme le permanganate de potassium, de préférence à l'aide de peroxyde d'hydrogène en milieu basique.

22. Procédé selon l'une des revendications 1 à 21, **caractérisé en ce que** ledit procédé est complété par l'isolement par distillation en présence d'une base du caprolactame à partir de sa solution aqueuse ayant subi les différentes étapes de purification précédentes.

## Claims

1. Process for purifying a lactam obtained from the cyclizing vapour-phase hydrolysis of an aliphatic aminonitrile, **characterized in that**, without prior distillation of the lactam and after most of the ammonia contained therein has been removed, the said lactam is subjected to a liquid/liquid extraction using a solvent comprising a solvent of acidic nature and/or the said lactam is placed in contact with a cation-exchange resin, and **in that** the lactam used is chosen from those obtained by cyclizing vapour-phase hydrolysis of an aliphatic aminonitrile of general formula (I):
N=C-R-NH₂ (I)
in which R represents an alkylene radical containing from 3 to 12 carbon atoms

2. Process according to claim 1, **characterized in that** the lactam used is chosen from those which are obtained by cyclizing vapour-phase hydrolysis of an aliphatic aminonitrile of general formula (I):
N=C-R-NH₂ (I)
in which R represents a linear alkylene radical containing 3, 4, 5 or 9 carbon atoms.

3. Process according to either of claims 1 and 2,
**characterized in that** the lactam preferentially used is caprolactam, prepared from 6-aminocapronitrile.

4. Process according to one of claims 1 to 3, **characterized in that** the lactam to be purified is in the form of an aqueous solution whose lactam concentration is from 20% to 80% on a weight for weight basis.

5. Process according to one of claims 1 to 4, **characterized in that** the unconverted aminonitrile represents up to 15% of the weight of the lactam and preferably from 0.1% to 10% of this weight.

6. Process according to one of claims 1 to 5, **characterized in that** the solvent of acidic nature used in the liquid/liquid extraction is an alkyl hydrogen phosphate and preferably a dialkyl hydrogen phosphate.

7. Process according to one of claims 1 to 6, **characterized in that** the solvent of acidic nature used in the liquid/liquid extraction is a dialkyl hydrogen phosphate in the formula of which the linear or branched and preferably identical alkyl groups contain from 1 to 12 carbon atoms.

8. Process according to one of claims 1 to 7, **characterized in that** the solvent of acidic nature used in the liquid/liquid extraction is bis(2-ethylhexyl) hydrogen phosphate.

9. Process according to one of claims 1 to 8, **characterized in that** the solvent of acidic nature is used as a mixture with another organic liquid, referred to as a diluent, the solubility of the lactam in the diluent being less than or equal to 200 grams per litre at 25°C and preferably less than or equal to 100 grams per litre.

10. Process according to one of claims 1 to 9, **characterized in that** the proportion of diluent in the liquid used to carry out the liquid/liquid extraction in the process of the invention ranges from 0% to 80% and preferably from 10% to 60% on a weight for weight basis.

11. Process according to one of claims 1 to 10, **characterized in that** the volume ratio between the aqueous lactam solution subjected to the extraction and the extraction solvent ranges between 1/5 and 5/1 and preferably between 2/1 and 1/2.

12. Process according to one of claims 1 to 11, **characterized in that** the temperature at which the extraction is carried out is between 10°C and 90°C and is preferably from 20°C to 80°C.

13. Process according to one of claims 1 to 5, **characterized in that** the cation-exchange resin is chosen from polymeric resins containing functions of acidic nature, preferably from resins bearing sulphonic acid or carboxylic acid functions, and polymeric resins of complexing nature, preferably containing imidoacetic or aminophosphonic functions.

14. Process according to claim 13, **characterized in that** the cation-exchange resin is chosen from cation-exchange resins containing sulphonic acid functions and containing a macroporous structure.

15. Process according to either of claims 13 and 14, **characterized in that** the lactam solution treated with a cation-exchange resin comprises from 10% to 90% of dissolved compounds by weight relative to the weight of the solution, and preferably from 20% to 80%.

16. Process according to one of claims 13 to 15, **characterized in that** the treatment on resin is carried out in a column and **in that** it comprises a cycle composed of an adsorption step, a step of rinsing after adsorption, a step of regeneration of the bed by placing in contact with a solution of a protic inorganic acid, and a step of rinsing after elution of the regeneration flow.

17. Process according to one of claims 13 to 16, **characterized in that** the regeneration of the resin is carried out using a solution of protic inorganic acid such as sulphuric acid, nitric acid or hydrochloric acid.

18. Process according to one of claims 1 to 17, **characterized in that** the liquid/liquid extraction step and/or the step of passage over acidic resin is preceded by or is combined with a hydrogenation step.

19. Process according to claim 18, **characterized in that** the hydrogenation step is carried out in the presence of a catalyst chosen from catalysts based on Raney nickel and/or on Raney cobalt, preferentially containing a doping element chosen from the elements of groups IVb, VIb, VIIb and VIII of the Periodic Table or catalysts consisting of a metal from group VIII of the Periodic Table of the Elements, such as ruthenium, rhodium, iridium, osmium, platinum, palladium, nickel or cobalt, deposited on a support.

20. Process according to one of claims 1 to 19, **characterized in that** the liquid/liquid extraction step and/or the step of passage over acidic resin is preceded or followed by an oxidation step.

21. Process according to claim 20, **characterized in that** the oxidation step is after the liquid/liquid extraction step and/or the step of passage over acidic resin and is carried out using hydrogen peroxide, ozone or oxidizing salts such as potassium permanganate, preferably using hydrogen peroxide in basic medium.

22. Process according to one of claims 1 to 21, **characterized in that** the said process is completed by isolation by distillation, in the presence of a base, of the caprolactam from its aqueous solution which has undergone the various purification steps above.

## Patentansprüche

1. Verfahren zur Reinigung eines Lactams, das aus der cyclisierenden Hydrolyse eines aliphatischen Aminonitrils in der Dampfphase stammt, **dadurch gekennzeichnet, daß** man das Lactam ohne vorherige Destillation und nachdem der Hauptteil des Ammoniaks, den es enthält, entfernt wurde, einer Flüssig-Flüssig-Extraktion mit Hilfe eines Lösungsmittels, das ein Lösungsmittel mit saurem Charakter umfaßt, unterzieht und/oder man das Lactam mit einem Kationenaustauscherharz in Kontakt. bringt und daß das verwendete Lactam aus denen ausgewählt wird, die durch cyclisierende Hydrolyse eines aliphatischen Aminonitrils der allgemeinen Formel (I) in der Dampfphase erhalten werden:
N≡C-R-NH₂ (I)
worin R eine Alkylengruppe mit 3 bis 12 Kohlenstoffatomen darstellt.

2. Verfahren nach Anspruch 1, **dadurch gekennzeichnet, daß** das verwendete Lactam unter denen ausgewählt wird, die durch cyclisierende Hydrolyse eines aliphatischen Aminonitrils der allgemeinen Formel (I) in der Dampfphase erhalten werden:
N≡C-R-NH₂ (I)
worin R eine lineare Alkylengruppe mit 3, 4, 5 oder 9 Kohlenstoffatomen darstellt.

3. Verfahren nach Anspruch 1 oder Anspruch 2, **dadurch gekennzeichnet, daß** das Lactam, das vorzugsweise verwendet wird, das Caprolactam ist, das ausgehend von 6-Aminocapronitril hergestellt wird.

4. Verfahren nach einem der Ansprüche 1 bis 3, **dadurch gekennzeichnet, daß** das zu reinigende Lactam in Form einer wäßrigen Lösung vorliegt, deren Konzentration an Lactam 20 Gew.-% bis 80 Gew.-% ist.

5. Verfahren nach einem der Ansprüche 1 bis 4, **dadurch gekennzeichnet, daß** das nicht umgewandelte Aminonitril bis zu 15 % des Gewichts des Lactams und vorzugsweise 0,1 bis 10 % dieses Gewichts bildet.

6. Verfahren nach einem der Ansprüche 1 bis 5, **dadurch gekennzeichnet, daß** das Lösungsmittel mit saurem Charakter, das in der Flüssig-Flüssig-Extraktion verwendet wird, ein Alkylhydrogenphosphat und vorzugsweise ein Dialkylhydrogenphosphat ist.

7. Verfahren nach einem der Ansprüche 1 bis 6, **dadurch gekennzeichnet, daß** das Lösungsmittel mit saurem Charakter, das in der Flüssig-Flüssig-Extraktion verwendet wird, ein Dialkylhydrogenphosphat ist, in dessen Formel die Alkylgruppen, die linear oder verzweigt und vorzugsweise identisch sind, 1 bis 12 Kohlenstoffatome haben.

8. Verfahren nach einem der Ansprüche 1 bis 7, **dadurch gekennzeichnet, daß** das Lösungsmittel mit saurem Charakter, das in der Flüssig-Flüssig-Extraktion verwendet wird, Di(2-ethylhexyl)-hydrogenphosphat ist.

9. Verfahren nach einem der Ansprüche 1 bis 8, **dadurch gekennzeichnet, daß** das Lösungsmittel mit saurem Charakter im Gemisch mit einer anderen organischen Flüssigkeit, die Verdünnungsmittel genannt wird, verwendet wird, wobei die Löslichkeit des Lactams in dem Verdünnungsmittel bei 25 °C 200 Gramm pro Liter und weniger und vorzugsweise 100 Gramm pro Liter oder weniger ist.

10. Verfahren nach einem der Ansprüche 1 bis 9, **dadurch gekennzeichnet, daß** der Anteil des Verdünnungsmittels in der Flüssigkeit, die zur Durchführung der Flüssig-Flüssig-Extraktion des erfindungsgemäßen Verfahrens verwendet wird, von 0 Gew.-% bis 80 Gew.-% und vorzugsweise von 10 Gew.-% bis 60 Gew.-% variiert.

11. Verfahren nach einem der Ansprüche 1 bis 10, **dadurch gekennzeichnet, daß** das Volumenverhältnis zwischen der wäßrigen Lösung von Lactam, die der Extraktion unterzogen wird, und dem Extraktionslösungsmittel zwischen 1/5 und 5/1 und vorzugsweise zwischen 2/1 und 1/2 variiert.

12. Verfahren nach einem der Ansprüche 1 bis 11, **dadurch gekennzeichnet, daß** die Temperatur, bei der die Extraktion durchgeführt wird, zwischen 10 °C und 90 °C liegt und vorzugsweise 20 °C bis 80 °C ist.

13. Verfahren nach einem der Ansprüche 1 bis 5, **dadurch gekennzeichnet, daß** das Kationenaustauscherharz unter den Polymerharzen, die Funktionen mit saurem Charakter tragen, vorzugsweise unter den Harzen, die Sulfonsäurefunktionen oder Carbonsäurefunktionen tragen, und den Polymerharzen mit komplexierendem Charakter, die vorzugsweise Imidoessigsäure oder Aminophosphonsäurefunktionen tragen, ausgewählt wird.

14. Verfahren nach Anspruch 13, **dadurch gekennzeichnet, daß** das Kationenaustauscherharz unter den Kationenaustauscherharzen mit Sulfonsäurefunktionen und mit makroporöser Struktur ausgewählt wird.

15. Verfahren nach Anspruch 13 oder Anspruch 14, **dadurch gekennzeichnet, daß** die Lactamlösung, die mit dem Kationenaustauscherharz behandelt wurde, 10 Gew.-% bis 90 Gew.-% gelöste Verbindungen, bezogen auf das Gewicht der Lösung, und vorzugsweise 20 % bis 80 % umfaßt.

16. Verfahren nach einem der Ansprüche 13 bis 15, **dadurch gekennzeichnet, daß** die Behandlung auf Harz in einer Säule durchgeführt wird und daß sie einen Zyklus umfaßt, der aus einer Adsorptionsstufe, einer Waschstufe nach Adsorption, einer Regenerationsstufe des Betts durch Inkontaktbringen mit einer Lösung einer Protonenmineralsäure und einer Waschstufe nach Elution des Regenerationsflusses besteht.

17. Verfahren nach einem der Ansprüche 13 bis 16, **dadurch gekennzeichnet, daß** die Regeneration des Harzes mit Hilfe einer Lösung einer Protonenmineralsäure wie Schwefelsäure, Salpetersäure, Salzsäure durchgeführt wird.

18. Verfahren nach einem der Ansprüche 1 bis 17, **dadurch gekennzeichnet, daß** der Stufe der Flüssig-Flüssig-Extraktion und/oder der Stufe des Leitens über saures Harz eine Hydrierungsstufe vorausgeht oder die genannten Stufen durch eine Hydrierung vervollständigt werden.

19. Verfahren nach Anspruch 18, **dadurch gekennzeichnet, daß** die Hydrierungsstufe in Gegenwart eines Katalysators, ausgewählt unter den Katalysatoren auf der Basis von Raney-Nickel und/oder Raney-Cobalt, die vorzugsweise ein Dotierungselement enthalten, das aus den Gruppen IVb, VIb, VIIb und VIII des Periodensystems ausgewählt ist, oder den Katalysatoren, die durch ein Metall der Gruppe VIII des Periodensystems der Elemente gebildet werden, wie Ruthenium, Rhodium, Iridium, Osmium, Platin, Palladium, Nickel oder Cobalt, das auf einem Träger abgeschieden ist, durchgeführt wird.

20. Verfahren nach einem der Ansprüche 1 bis 19, **dadurch gekennzeichnet, daß** der Stufe der Flüssig-Flüssig-Extraktion und/oder der Stufe des Leitens über ein saures Harz eine Oxidationsstufe vorausgeht oder sich anschließt.

21. Verfahren nach Anspruch 20, **dadurch gekennzeichnet, daß** die Oxidationsstufe nach der Stufe der Flüssig-Flüssig-Extraktion und/oder der Stufe des Leitens über saures Harz ist und mit Hilfe von Wasserstoffperoxid, Ozon oder oxidierenden Salzen wie Kaliumpermanganat, vorzugsweise mit Hilfe von Wasserstoffperoxid in basischem Milieu durchgeführt wird.

22. Verfahren nach einem der Ansprüche 1 bis 21, **dadurch gekennzeichnet, daß** das Verfahren durch die Isolierung des Caprolactams durch Destillation in Gegenwart einer Base, ausgehend von seiner wäßrigen Lösung, die verschiedenen vorangehenden Reinigungsstufen unterworfen worden war, vervollständigt wird.
